# EUROPEAN PATENT APPLICATION

(11) **EP 3 792 283 A1**
(43) Date of publication of application: **17.03.2021**
(21) Application number: 19197510.1
(22) Date of filing: 16.09.2019
(51) Int. Cl.: C07K 16/28, C07K 16/30, C07K 16/46, A61K 39/00

(54) **TREATMENT OF CANCER COMPRISING ADMINISTRATION OF VGAMMA9VDELTA2 T CELL RECEPTOR BINDING ANTIBODIES**

(71) Applicant: Lava Therapeutics B.V., 5223 DE' s-Hertogenbosch (NL)
(72) Inventor: ROOVERS, Robertus Cornelis, 3584 CM Utrecht (NL); HORBACH, Gerard Joseph Maria Jean, 5345 RR Oss (NL); RIEDL, Thilo Alexander, 3584 CM Utrecht (NL); VAN DER VLIET, Johannes Jelle, 1081 HV Amsterdam (NL); PARREN, Paul Willem Henri Ida, 3584 CM Utrecht (NL)
(74) Representative: Nederlandsch Octrooibureau

(57) **Abstract**

The invention relates to methods for the treatment of cancer. In particular, the invention relates to methods for the treatment of cancer, comprising administration of a high dose of an antibody capable of binding a human Vγ9Vδ2 T cell receptor.

SEQUENCE LISTING

<110>  LAVA Therapeutics

<120>  Treatment of cancer comprising administration of Vgamma9Vdelta2 T
       cell receptor binding antibodies

<130>  P6088266EP

<160>  23

<170>  PatentIn version 3.5

<210>  1
<211>  5
<212>  PRT
<213>  Artificial Sequence

<220>
<223>  antibody sequence

<400>  1

Asn Tyr Ala Met Gly
1               5


<210>  2
<211>  17
<212>  PRT
<213>  Artificial Sequence

<220>
<223>  antibody sequence

<400>  2

Ala Ile Ser Trp Ser Gly Gly Ser Thr Ser Tyr Ala Asp Ser Val Lys
1               5                   10                  15


Gly




<210>  3
<211>  21
<212>  PRT
<213>  Artificial Sequence

<220>
<223>  antibody sequence

<400>  3

Gln Phe Ser Gly Ala Asp Tyr Gly Phe Gly Arg Leu Gly Ile Arg Gly
1               5                   10                  15


Tyr Glu Tyr Asp Tyr
            20

<210>  4
<211>  5
<212>  PRT
<213>  Artificial Sequence

<220>
<223>  antibody sequence

<400>  4

Asn Tyr Gly Met Gly
1               5


<210>  5
<211>  17
<212>  PRT
<213>  Artificial Sequence

<220>
<223>  antibody sequence

<400>  5

Gly Ile Ser Trp Ser Gly Gly Ser Thr Asp Tyr Ala Asp Ser Val Lys
1               5                   10                  15


Gly


<210>  6
<211>  17
<212>  PRT
<213>  Artificial Sequence

<220>
<223>  antibody sequence

<400>  6

Val Phe Ser Gly Ala Glu Thr Ala Tyr Tyr Pro Ser Asp Asp Tyr Asp
1               5                   10                  15


Tyr


<210>  7
<211>  130
<212>  PRT
<213>  Artificial Sequence

<220>
<223>  antibody sequence

<400>  7

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Pro Phe Ser Asn Tyr
            20                  25                  30

Ala Met Gly Trp Phe Arg Gln Ala Pro Gly Lys Glu Arg Glu Phe Val
        35                  40                  45

Ala Ala Ile Ser Trp Ser Gly Gly Ser Thr Ser Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Pro Lys Pro Glu Asp Thr Ala Ile Tyr Tyr Cys
                85                  90                  95

Ala Ala Gln Phe Ser Gly Ala Asp Tyr Gly Phe Gly Arg Leu Gly Ile
            100                 105                 110

Arg Gly Tyr Glu Tyr Asp Tyr Trp Gly Gln Gly Thr Gln Val Thr Val
        115                 120                 125

Ser Ser
    130

<210> 8
<211> 130
<212> PRT
<213> Artificial Sequence

<220>
<223> antibody sequence

<400> 8

Glu Val Gln Leu Leu Glu Ser Gly Gly Gly Ser Val Gln Pro Gly Gly
1               5                   10                  15

Ser Leu Arg Leu Ser Cys Ala Ala Ser Gly Arg Pro Phe Ser Asn Tyr
            20                  25                  30

Ala Met Ser Trp Phe Arg Gln Ala Pro Gly Lys Glu Arg Glu Phe Val
        35                  40                  45

Ser Ala Ile Ser Trp Ser Gly Gly Ser Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Gln Phe Ser Gly Ala Asp Tyr Gly Phe Gly Arg Leu Gly Ile
            100                 105                 110

Arg Gly Tyr Glu Tyr Asp Tyr Trp Gly Gln Gly Thr Gln Val Thr Val
        115                 120                 125

Ser Ser
    130

<210>  9
<211>  130
<212>  PRT
<213>  Artificial Sequence

<220>
<223>  antibody sequence

<400>  9

Glu Val Gln Leu Leu Glu Ser Gly Gly Gly Leu Val Gln Pro Gly Gly
1               5                   10                  15

Ser Leu Arg Leu Ser Cys Ala Ala Ser Gly Arg Pro Phe Ser Asn Tyr
            20                  25                  30

Ala Met Ser Trp Phe Arg Gln Ala Pro Gly Lys Glu Arg Glu Phe Val
        35                  40                  45

Ser Ala Ile Ser Trp Ser Gly Gly Ser Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Gln Phe Ser Gly Ala Asp Tyr Gly Phe Gly Arg Leu Gly Ile
            100                 105                 110

Arg Gly Tyr Glu Tyr Asp Tyr Trp Gly Gln Gly Thr Leu Val Thr Val
        115                 120                 125

Ser Ser
    130

<210>  10

<211>  126
<212>  PRT
<213>  Artificial Sequence

<220>
<223>  antibody sequence

<400>  10

Glu Val Gln Leu Val Glu Ser Gly Gly Gly Leu Val Gln Ala Gly Gly
1               5                   10                  15

Ser Leu Arg Leu Ser Cys Ala Ala Ser Gly Arg Pro Phe Ser Asn Tyr
            20                  25                  30

Gly Met Gly Trp Phe Arg Gln Ala Pro Gly Lys Lys Arg Glu Phe Val
        35                  40                  45

Ala Gly Ile Ser Trp Ser Gly Gly Ser Thr Asp Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Val Phe Ser Gly Ala Glu Thr Ala Tyr Tyr Pro Ser Asp Asp
            100                 105                 110

Tyr Asp Tyr Trp Gly Gln Gly Thr Gln Val Thr Val Ser Ser
        115                 120                 125


<210>  11
<211>  5
<212>  PRT
<213>  Artificial Sequence

<220>
<223>  linker sequence

<400>  11

Gly Gly Gly Gly Ser
1               5


<210>  12
<211>  5
<212>  PRT
<213>  Artificial Sequence

<220>
<223>  antibody sequence

<400>  12

Ser Tyr Gly Met Gly
1               5


<210>  13
<211>  17
<212>  PRT
<213>  Artificial Sequence

<220>
<223>  antibody sequence

<400>  13

Gly Ile Ser Trp Arg Gly Asp Ser Thr Gly Tyr Ala Asp Ser Val Lys
1               5                   10                  15


Gly




<210>  14
<211>  15
<212>  PRT
<213>  Artificial Sequence

<220>
<223>  antibody sequence

<400>  14

Ala Ala Gly Ser Ala Trp Tyr Gly Thr Leu Tyr Glu Tyr Asp Tyr
1               5                   10                  15


<210>  15
<211>  124
<212>  PRT
<213>  Artificial Sequence

<220>
<223>  antibody sequence

<400>  15

Glu Val Gln Leu Val Glu Ser Gly Gly Gly Ser Val Gln Thr Gly Gly
1               5                   10                  15


Ser Leu Arg Leu Thr Cys Ala Ala Ser Gly Arg Thr Ser Arg Ser Tyr
            20                  25                  30


Gly Met Gly Trp Phe Arg Gln Ala Pro Gly Lys Glu Arg Glu Phe Val
        35                  40                  45


Ser Gly Ile Ser Trp Arg Gly Asp Ser Thr Gly Tyr Ala Asp Ser Val
    50                  55                  60

Lys Gly Arg Phe Thr Ile Ser Arg Asp Asn Ala Lys Asn Thr Val Asp
65                  70                  75                  80

Leu Gln Met Asn Ser Leu Lys Pro Glu Asp Thr Ala Ile Tyr Tyr Cys
                85                  90                  95

Ala Ala Ala Ala Gly Ser Ala Trp Tyr Gly Thr Leu Tyr Glu Tyr Asp
            100                 105                 110

Tyr Trp Gly Gln Gly Thr Gln Val Thr Val Ser Ser
        115                 120

<210>  16
<211>  259
<212>  PRT
<213>  Artificial Sequence

<220>
<223>  antibody sequence

<400>  16

Glu Val Gln Leu Val Glu Ser Gly Gly Gly Ser Val Gln Thr Gly Gly
1               5                   10                  15

Ser Leu Arg Leu Thr Cys Ala Ala Ser Gly Arg Thr Ser Arg Ser Tyr
            20                  25                  30

Gly Met Gly Trp Phe Arg Gln Ala Pro Gly Lys Glu Arg Glu Phe Val
        35                  40                  45

Ser Gly Ile Ser Trp Arg Gly Asp Ser Thr Gly Tyr Ala Asp Ser Val
    50                  55                  60

Lys Gly Arg Phe Thr Ile Ser Arg Asp Asn Ala Lys Asn Thr Val Asp
65                  70                  75                  80

Leu Gln Met Asn Ser Leu Lys Pro Glu Asp Thr Ala Ile Tyr Tyr Cys
                85                  90                  95

Ala Ala Ala Ala Gly Ser Ala Trp Tyr Gly Thr Leu Tyr Glu Tyr Asp
            100                 105                 110

Tyr Trp Gly Gln Gly Thr Gln Val Thr Val Ser Ser Gly Gly Gly Gly
        115                 120                 125

Ser Glu Val Gln Leu Leu Glu Ser Gly Gly Gly Ser Val Gln Pro Gly
    130                 135                 140

Gly Ser Leu Arg Leu Ser Cys Ala Ala Ser Gly Arg Pro Phe Ser Asn
145                 150                 155                 160

Tyr Ala Met Ser Trp Phe Arg Gln Ala Pro Gly Lys Glu Arg Glu Phe
                165                 170                 175

Val Ser Ala Ile Ser Trp Ser Gly Gly Ser Thr Ser Tyr Ala Asp Ser
            180                 185                 190

Val Lys Gly Arg Phe Thr Ile Ser Arg Asp Asn Ser Lys Asn Thr Leu
        195                 200                 205

Tyr Leu Gln Met Asn Ser Leu Arg Ala Glu Asp Thr Ala Val Tyr Tyr
    210                 215                 220

Cys Ala Ala Gln Phe Ser Gly Ala Asp Tyr Gly Phe Gly Arg Leu Gly
225                 230                 235                 240

Ile Arg Gly Tyr Glu Tyr Asp Tyr Trp Gly Gln Gly Thr Gln Val Thr
                245                 250                 255

Val Ser Ser

<210>  17
<211>  5
<212>  PRT
<213>  Artificial Sequence

<220>
<223>  antibody sequence

<400>  17

Asp Asn Val Met Gly
1               5

<210>  18
<211>  16
<212>  PRT
<213>  Artificial Sequence

<220>
<223>  antibody sequence

<400>  18

Thr Ile Arg Thr Gly Gly Ser Thr Asn Tyr Ala Asp Ser Val Lys Gly
1               5                   10                  15

<210>  19
<211>  11
<212>  PRT

<213>  Artificial Sequence

<220>
<223>  antibody sequence

<400>  19

Thr Ile Pro Val Pro Ser Thr Pro Tyr Asp Tyr
1               5                   10


<210>  20
<211>  119
<212>  PRT
<213>  Artificial Sequence

<220>
<223>  antibody sequence

<400>  20

Glu Val Gln Leu Val Glu Ser Gly Gly Gly Leu Val Gln Ala Gly Gly
1               5                   10                  15


Ser Leu Arg Leu Ser Cys Ala Ala Ser Gly Ser Met Phe Ser Asp Asn
            20                  25                  30


Val Met Gly Trp Tyr Arg Gln Ala Pro Gly Lys Gln Arg Glu Leu Val
        35                  40                  45


Ala Thr Ile Arg Thr Gly Gly Ser Thr Asn Tyr Ala Asp Ser Val Lys
    50                  55                  60


Gly Arg Phe Thr Ile Ser Arg Asp Asn Ala Lys Asn Thr Val Tyr Leu
65                  70                  75                  80


Gln Met Asn Ser Leu Lys Pro Glu Asp Thr Ala Val Tyr Tyr Cys Arg
                85                  90                  95


His Thr Ile Pro Val Pro Ser Thr Pro Tyr Asp Tyr Trp Gly Gln Gly
            100                 105                 110


Thr Gln Val Thr Val Ser Ser
        115


<210>  21
<211>  254
<212>  PRT
<213>  Artificial Sequence

<220>
<223>  antibody sequence

<400>  21

Glu Val Gln Leu Val Glu Ser Gly Gly Gly Leu Val Gln Ala Gly Gly
1               5                   10                  15

Ser Leu Arg Leu Ser Cys Ala Ala Ser Gly Ser Met Phe Ser Asp Asn
            20                  25                  30

Val Met Gly Trp Tyr Arg Gln Ala Pro Gly Lys Gln Arg Glu Leu Val
        35                  40                  45

Ala Thr Ile Arg Thr Gly Gly Ser Thr Asn Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys Arg
                85                  90                  95

His Thr Ile Pro Val Pro Ser Thr Pro Tyr Asp Tyr Trp Gly Gln Gly
            100                 105                 110

Thr Gln Val Thr Val Ser Ser Gly Gly Gly Gly Ser Glu Val Gln Leu
        115                 120                 125

Leu Glu Ser Gly Gly Gly Ser Val Gln Pro Gly Gly Ser Leu Arg Leu
    130                 135                 140

Ser Cys Ala Ala Ser Gly Arg Pro Phe Ser Asn Tyr Ala Met Ser Trp
145                 150                 155                 160

Phe Arg Gln Ala Pro Gly Lys Glu Arg Glu Phe Val Ser Ala Ile Ser
                165                 170                 175

Trp Ser Gly Gly Ser Thr Ser Tyr Ala Asp Ser Val Lys Gly Arg Phe
            180                 185                 190

Thr Ile Ser Arg Asp Asn Ser Lys Asn Thr Leu Tyr Leu Gln Met Asn
        195                 200                 205

Ser Leu Arg Ala Glu Asp Thr Ala Val Tyr Tyr Cys Ala Ala Gln Phe
    210                 215                 220

Ser Gly Ala Asp Tyr Gly Phe Gly Arg Leu Gly Ile Arg Gly Tyr Glu
225                 230                 235                 240

Tyr Asp Tyr Trp Gly Gln Gly Thr Gln Val Thr Val Ser Ser
                245                 250

<210>  22
<211>  20
<212>  PRT
<213>  Artificial Sequence

<220>
<223>  linker sequence

<400>  22

Gly Gly Gly Gly Ser Gly Gly Gly Gly Ser Gly Gly Gly Gly Ser Gly
1               5                   10                  15

Gly Gly Gly Ser
            20

<210>  23
<211>  124
<212>  PRT
<213>  Artificial Sequence

<220>
<223>  antibody sequence

<400>  23

Glu Val Gln Leu Val Glu Ser Gly Gly Gly Ser Val Gln Pro Gly Gly
1               5                   10                  15

Ser Leu Arg Leu Ser Cys Ala Ala Ser Gly Arg Thr Ser Arg Ser Tyr
            20                  25                  30

Gly Met Ser Trp Phe Arg Gln Ala Pro Gly Lys Glu Arg Glu Phe Val
        35                  40                  45

Ser Gly Ile Ser Trp Arg Gly Asp Ser Thr Gly Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Ala Ala Gly Ser Ala Trp Tyr Gly Thr Leu Tyr Glu Tyr Asp
            100                 105                 110

Tyr Trp Gly Gln Gly Thr Leu Val Thr Val Ser Ser
        115                 120

## Description

### Field of the invention

The present invention relates to novel methods for the treatment of cancer, in particular to methods for the treatment of cancer that comprise administration of an antibody that binds a human Vγ9Vδ2 T cell receptor.

### Background of the invention

Bi-specific T-cell engagers (bsTCEs) are bispecific antibodies directed against a constant-component of the T-cell/CD3 complex and a tumor-associated antigen. Under physiological conditions, CD8 T-cells only direct their cytotoxic activity towards cells expressing major histocompatibility class (MHC) I molecules loaded with peptides they recognize in the context of the T-cell receptor (TCR). Cancer cells have mechanisms to escape normal immune surveillance. By bypassing the normal TCR-MHC interaction to trigger T-cell activation, bsTCEs are not affected by similar escape mechanisms and can elicit a polyclonal T-cell response against cancer cells that express the tumor-associated antigen.

The interest in the use of bsTCEs for cancer treatment has been growing over recent years. However, along with growing experience in the clinical application and effectiveness of these potent immunotherapeutic agents came increasing awareness of inherent and potentially fatal adverse effects of antibodies having a CD3-binding arm. An example of such an adverse effect is the cytokine release syndrome (see e.g. Shimabukuro-Vornhagen et al (2018) J. ImmunoTher. Cancer, 6 (1):56). This cytokine release syndrome can be a dose-limiting side effect (Teachey et al. (2013) Blood, 121 (26): 5154). Severe and fatal toxicity of a CD3/EGFR bsTCE has been observed in cynomolgus monkeys at doses as low as 31 µg/kg/day and higher which corresponds to 0.5 nmol/kg/day or 0.02 nmol/kg/hr (Lutterbuese et al. (2010) PNAS 107(28): 12605). A CD3/EpCAM bsTCE was found to induce toxicities in human studies which precluded dose-escalation to potentially therapeutic levels (Kebenko et al. (2018) Oncoimmunology 7(8) e1450710). The maximal tolerated dose was a total dose of 24 µg (0.4 nmol) continuous infusion, which corresponds 0.005 nmol/kg/day or 0.0002 nmol/kg/hr for an average 80 kg patient.

There is therefore a need for improved methods of cancer treatment involving bsTCEs wherein efficacious doses can be given yet adverse effects are avoided. Instead of a CD3 binding arm, it has been proposed to specifically recruit NK cells (Hartmann et al. (1997) Blood, 89(6): 2042), iNKT Cells (Stirnemann et al. (2008) J. Clin. Invest., 118(3):994), gammadelta T cells (WO15156673), or CD8+ T cells (Koristka et al. (2015) Oncoimmunology, 4(3), e994441). However, only limited in vivo evidence is available.

Other strategies have been based on bsTCEs that have low-affinity CD3 binding (Moore et al. (2015) Tuning T cell affinity improves efficacy and safety of anti-CD38 × anti-CD3 bispecific antibodies in monkeys - a potential therapy for multiple myeloma. Blood, 126 (23): 1798). European patent EP2493503B1 describes a method for dosing of blinatumomab, a CD19/CD3 bsTCE, wherein undesired adverse events are reduced by adapting a patient to a low dose of the antibody prior to treatment with a therapeutic dose. Proposed doses of blinatumomab were between 1 and 60 µg/m²/day continuous infusion (which corresponds to about 2-100 µg/kg/day). The dose approved for blinatumomab amounts to 9 µg/day (days 1-7) and 28 µg/day on days 8-28 continuous infusion (continuing at 28 µg/day continuous infusion for following cycles for patients equal to or greater than 45 kg and 15 µg/m2/day (not exceeding at total dose of 28 µg) continuous infusion for patients less than 45 kg (blinatumomab, package insert)). The maximal blinatumomab dose therefore amounts to 5.6 µg/kg/day or 0.23 µg/kg/hr which corresponds to approximately 0.09 nmol/kg/day or 0.004 nmol/kg/hr.

### Summary of the invention

It has now surprisingly been found that bsTCE-like antibodies that bind a Vγ9Vδ2 T cell receptor can be dosed at unexpected high doses in primates without causing significant toxicity or adverse events. This surprising observation was made for several bsTCE-like antibodies binding to different tumor antigens, including a bsTCE-like antibody that binds EGFR, which is a tumor target also extensively found in healthy tissues.

Thus, in a first aspect, the invention relates to a method for the treatment of cancer, comprising administration of an antibody capable of binding to a human Vγ9Vδ2 T cell receptor to a human subject in need thereof, wherein the treatment regimen comprises one or more administrations of a dose of at least 2 nmol/kg/day.

In a further aspect, the invention relates to an antibody comprising an antigen-binding region capable of binding a human Vγ9Vδ2 T cell receptor for use in the treatment of cancer, wherein the treatment regimen comprises one or more administrations of a dose of at least 2 nmol/kg/day.

Further aspects and embodiments of the invention are described below.

### Brief description of the drawings

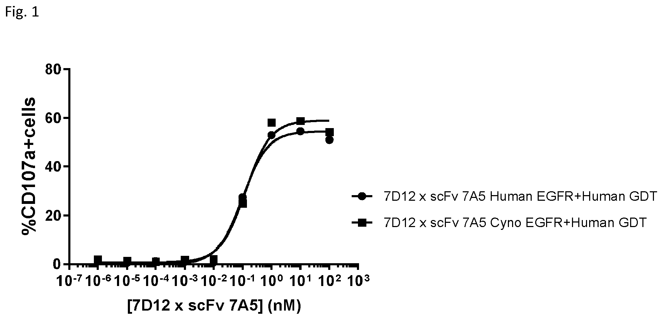

**Figure 1****:** 7D12 x scFv 7A5-mediated human Vγ9Vδ2 (GDT) cell degranulation (% of Vγ9Vδ2 cells positive for CD107a staining) dependent on either coated human-(circles) or cynomolgus (squares) EGFR. % of Vγ9Vδ2 cells positive for CD107A (Y-axis) is depicted as a function of the concentration of LAVA-043 used (X-axis).

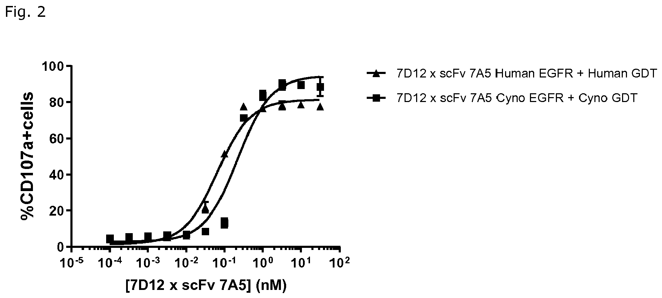

**Figure 2****:** 7D12 x scFv 7A5-mediated human Vγ9Vδ2cell degranulation (% of Vγ9Vδ 2cells positive for CD107a staining) dependent on coated human EGFR (circles) and 7D12 x scFv 7A5-mediated cynomolgus Vγ9Vδ2 cell degranulation dependent on coated cynomolgus EGFR (squares). % of Vγ9Vδ2 cells positive for CD107a (Y-axis) is depicted as a function of the concentration of antibody used (X-axis).

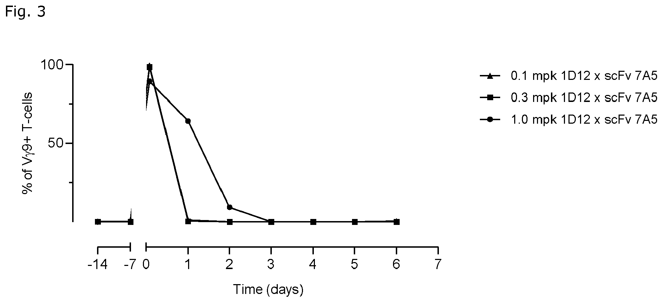

**Figure 3****:** Detection of VHH bound to Vy9 cells in cynomolgus monkey after a single iv infusion of 1D12 x scFv 7A5. The percentage of Vy9 cells that scored positive for VHH staining (Y-axis) was measured over time (X-axis) after the iv infusion. Three doses were used: 0.1mg/kg (triangles), 0.3mg/kg (squares) and 1.0mg/kg (circles).

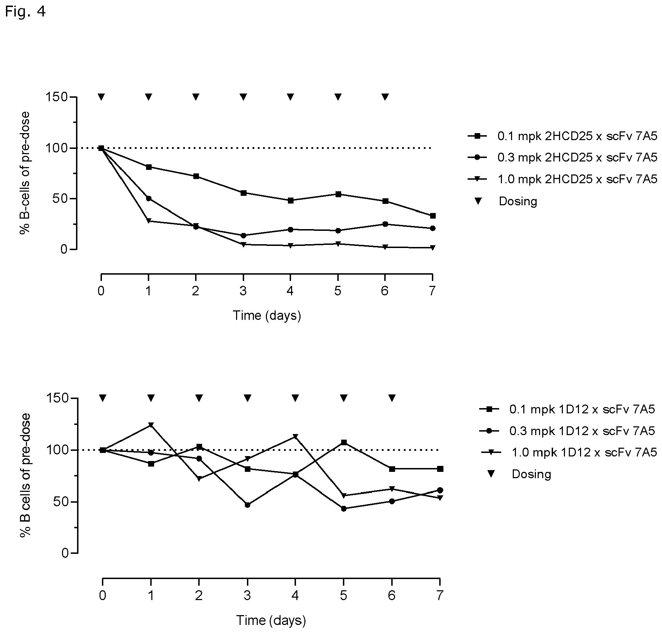

**Figure 4****:** 2HCD25 x scFv 7A5, but not 1D12 x scFv 7A5, causes a dose-dependent B cell depletion in cynomolgus monkey after repeated dose administration. The number of B cells (expressed as percentage of the number found before the start of dosing: Y-axis) was measured by flow cytometry over time (X-axis). Three doses were used. Squares: 0.1 mg/kg/day (mpk); circles: 0.3 mg/kg/day and triangles: 1.0 mg/kg/day. Large arrow heads indicate the timing of each dose given.

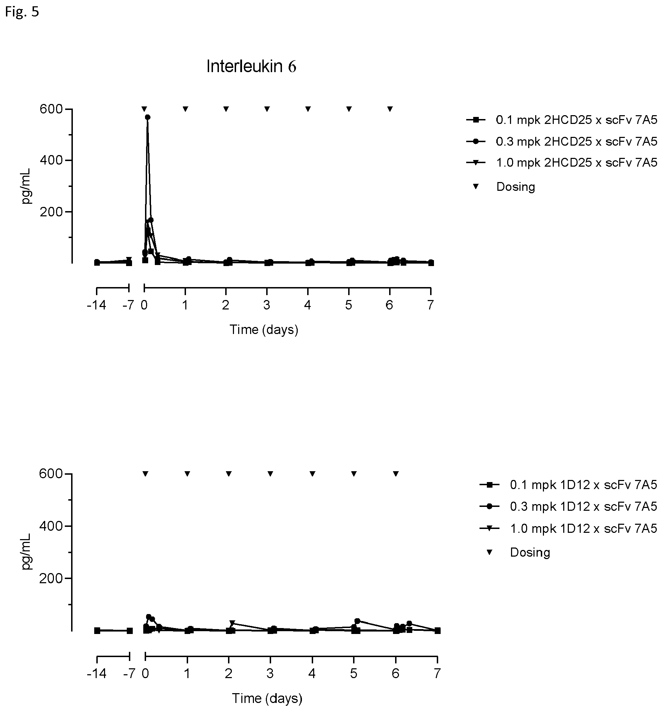

**Figure 5****:** 2HCD25 x scFv 7A5, but not 1D12 x scFv 7A5, causes a minor single dose-dependent cytokine release (peak) in the blood of cynomolgus monkey after repeated dose administration. The concentration of IL-6 (Y-axis) was determined in plasma of treated animals over time (X-axis). Top figure: animal that was treated with 2HCD25 x scFv 7A5; bottom figure: animal that was treated with 1D12 x scFv 7A5. Arrow heads indicate the timing of each dose given.

### Detailed description of the invention

### Definitions

An antibody "dose" of, for example, 1 nmol/kg/day means that 1 nmol of the antibody is administered across one day per kg of body weight. A daily dose may e.g. be given in a single administration, in several administrations or continuously across one day or part thereof, e.g. via an infusion which is allowed to proceed without interruption.

"Treatment" or "treating" refers to the administration of an effective amount of an antibody according to the present invention with the purpose of easing, ameliorating, arresting, eradicating (curing) or preventing symptoms or disease states. An "effective amount" refers to an amount effective, at dosages and for periods of time necessary, to achieve a desired therapeutic result. An effective amount is also one in which any toxic or detrimental effects of the antibody are outweighed by the therapeutically beneficial effects. Administration may be carried out by any suitable route, but will typically be parenteral, such as intravenous, intramuscular or subcutaneous.

The term "human Vδ2", when used herein, refers to the TRDV2 protein, T cell receptor delta variable 2 (UniProtKB - A0JD36 (A0JD36_HUMAN) gives an example of a Vδ2 sequence).

The term "human Vγ9", when used herein, refers to the TRGV9 protein, T cell receptor gamma variable 9 (UniProtKB - Q99603_HUMAN gives an example of a Vy9 sequence).

The term "antibody" is intended to refer to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The antigen-binding region (or antigen-binding domain) which interacts with an antigen may comprise variable regions of both the heavy and light chains of the immunoglobulin molecule or may be a single-domain antigen-binding region, e.g. a heavy chain variable region only. The constant regions of an antibody, if present, may mediate the binding of the immunoglobulin to host tissues or factors, including various cells of the immune system (such as effector cells and T cells) and components of the complement system such as C1q, the first component in the classical pathway of complement activation. In some embodiments, however, the Fc region of the antibody has been modified to become inert, "inert" means an Fc region which is at least not able to bind any Fcγ receptors, induce Fc-mediated cross-linking of FcRs, or induce FcR-mediated cross-linking of target antigens via two Fc regions of individual antibodies. In a further embodiment, the inert Fc region is in addition not able to bind C1q. In one embodiment, the antibody contains mutations at positions 234 and 235 (Canfield and Morrison (1991) J Exp Med 173:1483), e.g. a Leu to Phe mutation at position 234 and a Leu to Glu mutation at position 235. In another embodiment, the antibody contains a Leu to Ala mutation at position 234, a Leu to Ala mutation at position 235 and a Pro to Gly mutation at position 329. In another embodiment, the antibody contains a Leu to Phe mutation at position 234, a Leu to Glu mutation at position 235 and an Asp to Ala at position 265. In another embodiment, the antibody contains a Gly into Ala mutation at position 237 or a combination of mentioned mutations at 234, 235 and 237.

As indicated above, the term antibody as used herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include (i) a Fab' or Fab fragment, i.e. a monovalent fragment consisting of the VL, VH, CL and CH1 domains, or a monovalent antibody as described in WO2007059782; (ii) F(ab')2 fragments, i.e. bivalent fragments comprising two Fab fragments linked by a disulfide bridge at the hinge region; (iii) a Fd fragment consisting essentially of the VH and CH1 domains; and (iv) a Fv fragment consisting essentially of the VL and VH domains of a single arm of an antibody. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise indicated by context. Although such fragments are generally included within the meaning of antibody, they collectively and each independently are unique features of the present invention, exhibiting different biological properties and utility. The term antibody, unless specified otherwise, also includes polyclonal antibodies, monoclonal antibodies (mAbs), chimeric antibodies and humanized antibodies, and antibody fragments provided by any known technique, such as enzymatic cleavage, peptide synthesis, and recombinant techniques.

In some embodiments of the antibodies used in the invention, the first antigen-binding region or the antigen-binding region, or both, is a single domain antibody, such as a heavy chain-only antibody. Single domain antibodies (sdAb, also called Nanobody®, or VHH) are well known to the skilled person, see e.g. Hamers-Casterman et al. (1993) Nature 363:446, Roovers et al. (2007) Curr Opin Mol Ther 9:327 and Krah et al. (2016) Immunopharmacol Immunotoxicol 38:21. Single domain antibodies comprise a single CDR1, a single CDR2 and a single CDR3. Examples of single domain antibodies are variable fragments of heavy-chain-only antibodies, antibodies that naturally do not comprise light chains, single domain antibodies derived from conventional antibodies. Single domain antibodies may be derived from any species including mouse, human, camel, llama, shark, goat, rabbit, and cow. For example, naturally occurring VHH molecules can be derived from antibodies raised in Camelidae species, for example in camel, dromedary, alpaca llama and guanaco. Like a whole antibody, a single domain antibody is able to bind selectively to a specific antigen. Single domain antibodies may contain only the variable domain of an immunoglobulin chain, i.e. CDR1, CDR2 and CDR3 and framework regions.

The term "immunoglobulin" as used herein is intended to refer to a class of structurally related glycoproteins consisting of two pairs of polypeptide chains, one pair of light (L) chains and one pair of heavy (H) chains, all four potentially inter-connected by disulfide bonds. The term "immunoglobulin heavy chain", "heavy chain of an immunoglobulin" or "heavy chain" as used herein is intended to refer to one of the chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The heavy chain constant region further comprises a hinge region. Within the structure of the immunoglobulin (e.g. IgG), the two heavy chains are inter-connected via disulfide bonds in the hinge region. Equally to the heavy chains, each light chain is typically comprised of several regions; a light chain variable region (VL) and a light chain constant region (CL). Furthermore, the VH and VL regions may be subdivided into regions of hypervariability (or hypervariable regions which may be hypervariable in sequence and/or form structurally defined loops), also termed CDRs (complementarity determining regions), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Choitia and Lesk (1987) J. Mol. Biol. 196:901 or Kabat et al. (1991) Sequence of protein of immunological interest, fifth edition. NIH publication. Various methods for CDR determination and amino acid numbering can be compared on www.abysis.org (UCL). In addition, the term 'immunoglobulin' is also meant to include structurally related proteins consisting of single heavy chains paired via disulfide bonds, i.e. only containing an immunoglobulin heavy chain.

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) that is encoded by heavy chain constant region genes. Each heavy chain isotype can be combined with either a kappa (κ) or lambda (λ) light chain. An antibody used in the invention can possess any isotype.

The term "full-length antibody" when used herein, refers to an antibody which contains all heavy and light chain constant and variable domains corresponding to those that are normally found in a wild-type antibody of that isotype.

The term "chimeric antibody" refers to an antibody wherein the variable region is derived from a non-human species (e.g. derived from rodents) and the constant region is derived from a different species, such as human. Chimeric antibodies may be generated by genetic engineering. Chimeric monoclonal antibodies for therapeutic applications are developed to reduce antibody immunogenicity.

The term "humanized antibody" refers to a genetically engineered non-human antibody, which contains human antibody constant domains and non-human variable domains modified to contain a high level of sequence homology to human variable domains. This can be achieved by grafting of the six non-human antibody complementarity-determining regions (CDRs), which together form the antigen binding site, onto a homologous human acceptor framework region (FR). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and, optionally, fully human constant regions. Optionally, additional amino acid modifications, which are not necessarily back-mutations, may be introduced to obtain a humanized antibody with preferred characteristics, such as affinity and biochemical properties. Humanization of non-human therapeutic antibodies is performed to minimize its immunogenicity in man while such humanized antibodies at the same time maintain the specificity and binding affinity of the antibody of non-human origin. Humanization of heavy-chain only antibodies, or VHH domains, is achieved by grafting the three heavy-chain CDRs in a human framework while taking amino acid positions that adapt the molecule for proper solubility, stability and expression in the absence of a light chain into account (Vincke et al. JBC 284:3273-3284, 2009)

The term "multispecific antibody" refers to an antibody having specificities for at least two different, such as at least three, typically non-overlapping, epitopes. Such epitopes may be on the same or on different target antigens. If the epitopes are on different cell-bound targets, such targets may be on the same cell or different cells or cell types.

The term "bispecific antibody" refers to an antibody having specificities for two different, typically non-overlapping, epitopes. Such epitopes may be on the same or different targets. If the epitopes are on different cell-bound targets, such targets may be on the same cell or different cells or cell types.

Examples of different classes of bispecific antibodies include but are not limited to (i) IgG-like molecules with complementary, engineered CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab- fragments are fused together, fused to heavy-chain constant-domains, Fc-regions or parts thereof; and (vi) ScFv-and diabody-based and heavy chain antibodies (e.g., domain antibodies, Nanobodies®) wherein different single chain Fv molecules or different diabodies or different heavy-chain antibodies (e.g. domain antibodies, Nanobodies®) are fused to each other or to another protein or carrier molecule fused to heavy-chain constant-domains, Fc-regions or parts thereof.

Examples of IgG-like molecules with complementary CH3 domains molecules include but are not limited to the Triomab® (Trion Pharma/Fresenius Biotech), the Knobs-into-Holes (Genentech), CrossMAbs (Roche) and the electrostatically-matched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck,), mAb-Fv (Xencor), bivalent bispecific antibodies (Roche, WO2009080254) and DuoBody® molecules (Genmab).

Examples of recombinant IgG-like dual targeting molecules include but are not limited to Dual Targeting (DT)-Ig (GSK/Domantis, WO2009058383), Two-in-one Antibody (Genentech, Bostrom, et al 2009. Science 323, 1610-1614), Crosslinked Mabs (Karmanos Cancer Center), mAb2 (F-Star), ZybodiesTM (Zyngenia, LaFleur et al. MAbs. 2013 Mar-Apr;5(2):208-18), approaches with common light chain, κλBodies (NovImmune, WO2012023053) and CovX-body® (CovX/Pfizer, Doppalapudi, V.R., et al 2007. Bioorg. Med. Chem. Lett. 17,501-506).

Examples of IgG fusion molecules include but are not limited to Dual Variable Domain (DVD)-Ig (Abbott), Dual domain double head antibodies (Unilever; Sanofi Aventis), IgG-like Bispecific (ImClone/Eli Lilly, Lewis et al. Nat Biotechnol. 2014 Feb;32(2):191-8), Ts2Ab (MedImmune/AZ, Dimasi et al. J Mol Biol. 2009 Oct 30;393(3):672-92) and BsAb (Zymogenetics, WO2010111625), HERCULES (Biogen Idec), scFv fusion (Novartis), scFv fusion (Changzhou Adam Biotech Inc) and TvAb (Roche).

Examples of Fc fusion molecules include but are not limited to ScFv/Fc Fusions (Academic Institution, Pearce et al Biochem Mol Biol Int. 1997 Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock® (DNL) (ImmunoMedics), Bivalent Bispecific (Biotecnol) and Fab-Fv (UCB-Celltech).

Examples of ScFv-, diabody-based and domain antibodies include but are not limited to Bispecific T Cell Engager (BiTE®) (Micromet, Tandem Diabody (Tandab) (Affimed), Dual Affinity Retargeting Technology (DARTTM) (MacroGenics), Single-chain Diabody (Academic, Lawrence FEBS Lett. 1998 Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75), dual targeting nanobodies® (Ablynx, Hmila et al., FASEB J. 2010), dual targeting heavy chain only domain antibodies. An overview of bispecific antibody formats is provided in Labrijn et al. Nature Rev. Drug Discovery 2019 Aug;18(8):585-608.

In the context of antibody binding to an antigen, the terms "binds" or "specifically binds" refer to the binding of an antibody to a predetermined antigen or target (e.g. human Vδ2) to which binding typically is with an affinity corresponding to a K_{D} of about 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less, e.g. when determined using flow cytometry by incubating cells (e.g. Vγ9Vδ2 T cells) with a monovalent antibody and determining half maximum binding using FACS and a fluorescent antibody for detection. Alternatively, K_{D} values can be determined using by for instance surface plasmon resonance (SPR) technology in a BIAcore T100 instrument using the antigen as the ligand and the monovalent binding moiety or antibody as the analyte, or vice versa. Specific binding means that the antibody binds to the predetermined antigen with an affinity corresponding to a K_{D} that is at least ten-fold lower, such as at least 100-fold lower, for instance at least 1,000 fold lower, such as at least 10,000 fold lower, for instance at least 100,000 fold lower than its affinity for binding to a non-specific antigen (e.g., BSA, casein) other than the predetermined antigen or a closely-related antigen. The degree with which the affinity is lower is dependent on the K_{D} of the binding moiety or binding molecule, so that when the K_{D} of the binding moiety or binding molecule is very low (that is, the binding moiety or binding molecule is highly specific), then the degree with which the affinity for the antigen is lower than the affinity for a non-specific antigen may be at least 10,000-fold. The term "K_{D}" (M), as used herein, refers to the dissociation equilibrium constant of a particular interaction between the antigen and the binding moiety or binding molecule.

In the context of the present invention, "competition" or "able to compete" or "competes" refers to any detectably significant reduction in the propensity for a particular binding molecule (e.g. a Vδ2 antibody) to bind a particular binding partner (e.g. Vδ2) in the presence of another molecule (e.g. a different Vδ2 antibody) that binds the binding partner. Typically, competition means an at least about 25 percent reduction, such as an at least about 50 percent, e.g. an at least about 75 percent, such as an at least 90 percent reduction in binding, caused by the presence of another molecule, such as an antibody, as determined by, e.g., ELISA analysis or flow cytometry using sufficient amounts of the two or more competing molecules, e.g. antibodies. Additional methods for determining binding specificity by competitive inhibition may be found in for instance Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc, and Wiley InterScience N. Y., (1992, 1993), and Muller, Meth. Enzymol. 92, 589-601 (1983)). In one embodiment, the antibody used in the method of the present invention binds to the same epitope on Vδ2 as antibody 5C8 or 6H4. Methods for determining the epitope of a binding molecule, such as an antibody, are known in the art.

The terms "first" and "second" antigen-binding regions when used herein do not refer to their orientation / position in the antibody, i.e. it has no meaning with regard to the N- or C-terminus. The term "first" and "second" only serves to correctly and consistently refer to the two different antigen-binding regions in the claims and the description.

"Capable of binding a Vγ9Vδ2-TCR" means that the binding molecule can bind a Vγ9Vδ2-TCR, but does not exclude that the binding molecule binds to one of the separate subunits in the absence of the other subunit, i.e. to the Vy9 chain alone or to the Vδ2 chain alone. For example, antibody 5C8 is an antibody that binds the Vγ9Vδ2-TCR, but also binds the Vδ2 chain when the Vδ2 chain is expressed alone.

In some embodiments, the antibody used in the method of the invention is capable of activating human Vγ9Vδ2 T cells. The activation of the Vγ9Vδ2 T cells may be measured through gene-expression and/or (surface) marker expression (e.g., activation markers, such as CD25, CD69, or CD107a) and/or secretory protein (e.g., cytokines or chemokines) profiles. In a preferred embodiment, the antibody is able to induce activation (e.g. upregulation of CD69 and/or CD25 expression) resulting in degranulation marked by an increase in CD107a expression and/or cytokine production (e.g. TNFα, IFNγ) by Vγ9Vδ2 T cells. Preferably, the antibody used in the present invention is able to increase the number of cells positive for CD107a at least 1.5-fold, such as at least 2-fold, e.g. at least 5-fold.

"% sequence identity", when used herein, refers to the number of identical nucleotide or amino acid positions shared by different sequences (i.e., % identity = # of identical positions/total # of positions x 100), taking into account the number of gaps, and the length of each gap, which need to be introduced for optimal alignment. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4.

### Further aspects and embodiments of the invention

As described above, in a first aspect, the invention relates to a method for the treatment of cancer, comprising administration of an antibody capable of binding a human Vγ9Vδ2 T cell receptor to a human subject in need thereof, wherein the treatment regimen comprises one or more administrations of a dose of at least 2 nmol/kg/day. In one embodiment, the antibody binds monovalently to the Vγ9Vδ2 T cell receptor. If the antibody binds multivalently to the Vγ9Vδ2 T cell receptor, the antibody may be dosed at an equivalent lower dose, e.g. at least 1 nmol/kg/day for an antibody that binds bivalently to the Vγ9Vδ2 T cell receptor.

In one embodiment, the treatment regimen comprises one or more administrations of a dose of at least 2.5 nmol/kg/day, e.g. a dose of at least 5 nmol/kg/day, such as a dose of at least 10 nmol/kg/day, e.g. a dose of at least 25 nmol/kg/day, such as a dose of at least 50 nmol/kg/day, e.g. a dose of at least 100 nmol/kg/day.

In another embodiment, the treatment regimen comprises one or more administrations of a dose of at least 2 nmol/kg/day, such as at least 5, at least 10, at least 25, at least 50, at least 100 nmol/kg/day, but at most 10000 nmol/kg/day, e.g. at most 5000 nmol/kg/day, such as at most 1000 nmol/kg/day, e.g. at most 500 nmol/kg/day, such as at most 200 or at most 150 nmol/kg/day.

The administration may be performed using via any suitable route. In one embodiment, said administration is carried out intravenously over a time period of between 0.5 and 8 hours, such as between 1 and 8 hours, e.g. between 2 and 6 hours or between 1 and 4 hours.

In another embodiment, said administration is carried out subcutaneously or intramuscularly.

In one embodiment of the method of the invention, said dose is administered more than once. In a further embodiment, said dose is administered more than once and the time interval between each of the administrations is from 7 to 21 days, e.g. wherein administration is twice weekly, weekly or once every two weeks. The total duration of the treatment may e.g. be between two weeks and 12 months, such as between 2 and 6 months. Thus, e.g. in one embodiment, the method comprises administrations once per week, once per two weeks or once per three weeks over a period of between two-three weeks and 48 months, such as between 2 and 12 months, e.g. between 2 and 6 months. In a further embodiment, the method comprises administrations of a dose of between 10 and 500 nmol/kg/day once every two weeks over a period of between two weeks and 12 months, such as between 2 and 6 months. In another embodiment, one or more initial administrations below 2 nmol/kg/day, e.g. between 0.5-2 nmol/kg/day, are given before raising the dose to 2 nmol/kg/day for subsequent administrations.

In another embodiment the antibody dose is administered over a short period of time, such as 30 minutes, 1 hr, 2 hrs of 4 hrs. The treatment regimen then comprises one administration during the course of 0.5-4 hrs in a single day of at least 2 nmol/kg/hr, e.g. a dose of at least 5 nmol/kg/hr, such as a dose of at least 10 nmol/kg/hr, e.g. a dose of at least 25 nmol/kg/hr, such as a dose of at least 50 nmol/kg/hr, e.g. a dose of at least 100 nmol/kg/hr.

In another embodiment, the treatment regimen then comprises one administration during the course of 0.5-4 hrs in a single day of at least 2 nmol/kg/hr, such as at least 5, at least 10, at least 25, at least 50, at least 100 nmol/kg/hr, but at most 10000 nmol/kg/day, e.g. at most 5000 nmol/kg/day, such as at most 1000 nmol/kg/day, e.g. at most 500 nmol/kg/day, such as at most 200 or at most 150 nmol/kg/day.

In one embodiment of the method of the invention, the antibody capable of binding a human Vγ9Vδ2 T cell receptor is capable of activating human Vγ9Vδ2 T cells.

The activation of the Vγ9Vδ2 T cells may be measured through gene-expression and/or (surface) marker expression (e.g., activation markers, such as CD25, CD69, or CD107a) and/or secretory protein (e.g., cytokines or chemokines) profiles. In a preferred embodiment, the multispecific antibody is able to induce activation (e.g. upregulation of CD69 and/or CD25 expression) resulting in degranulation marked by an increase in CD107a expression, see Example herein) and cytokine production (e.g. TNFα, IFNγ) by Vγ9Vδ2 T cells. Preferably, an antibody used in the present invention is able to increase the number of cells positive for CD107a at least 1.5-fold, such as at least 2-fold, e.g. at least 5-fold, such as at least 10-fold, e.g. at least 25-fold when tested as described in the Examples herein.

In a further embodiment, the antibody capable of binding a human Vγ9Vδ2 T cell receptor comprises or consists of a single-domain antibody.
In a preferred embodiment, the antibody is a high affinity antibody, e.g. the antibody has a K_{D} for Vγ9Vδ2 T cell receptor binding of 10⁻⁸ M or less, such as 10⁻⁹ M or less or 10⁻¹⁰ M or less.

In one embodiment of the method of the invention, the antibody is capable of binding to human Vγ9.

In another embodiment of the method of the invention, the antibody is capable of binding to human Vδ2.

In a further embodiment of the method of the invention, the antibody competes for binding to human Vδ2 with an antibody having the sequence set forth in SEQ ID NO:8 or competes for binding to human Vδ2 with an antibody having the sequence set forth in SEQ ID NO:10, e.g. wherein the antibody binds the same epitope on human Vδ2 as an antibody having the sequence set forth in SEQ ID NO:8 or binds the same epitope on human Vδ2 as an antibody having the sequence set forth in SEQ ID NO:10.

In a further embodiment, the antibody comprises an antigen-binding region comprising the VH CDR1 sequence set forth in SEQ ID NO:1, the VH CDR2 sequence set forth in SEQ ID NO:2 and the VH CDR3 sequence set forth in SEQ ID NO:3 or comprises the VH CDR1 sequence set forth in SEQ ID NO:4, the VH CDR2 sequence set forth in SEQ ID NO:5 and the VH CDR3 sequence set forth in SEQ ID NO:6.

In a further embodiment, wherein the antibody comprises or consists of a sequence selected from the group consisting of: SEQ ID NO: 7, 8, 9 or 10.

In a preferred embodiment of the method of the invention, the antibody capable of binding a human Vγ9Vδ2 T cell receptor is a multispecific antibody having a (first) antigen-binding region capable of binding human Vγ9Vδ2 T cell receptor and a second antigen-binding region capable of binding a tumor antigen. Said antibody may comprise two or more antigen-binding specificities. In one embodiment, the multispecific antibody is a bispecific antibody.

In one embodiment, the second antigen-binding region of the multispecific antibody comprises or consists of a single-domain antibody. In one embodiment, the first antigen-binding region and the second antigen-binding region are covalently linked via a peptide linker, e.g. a peptide linker comprising or consisting of a Gly-Gly-Gly-Gly-Ser linker (SEQ ID NO:11).

In one embodiment of the method of the invention, the method is for the treatment of a solid tumor cancer and the antibody comprises a second antigen-binding region capable of binding a target expressed by solid tumors.

In one embodiment, the antibody comprises a second antigen-binding region capable of binding human Epidermal Growth Factor EGFR (HER1). In a further embodiment, the antibody comprises a second antigen-binding region capable of binding human EGFR and the method is for the treatment of a cancer selected from the group consisting of: lung cancer, such as non-small cell lung cancer, colorectal cancer, glioblastoma, hepatocellular carcinoma and squamous cell carcinoma of the head and neck.

In a further embodiment, the antibody comprises a second antigen-binding region comprising the VH CDR1 sequence set forth in SEQ ID NO:12, the VH CDR2 sequence set forth in SEQ ID NO:13 and the VH CDR3 sequence set forth in SEQ ID NO:14.

In an even further embodiment, the antibody comprises a second antigen-binding region comprising the sequence set forth in SEQ ID NO:15 or SEQ ID NO:23, preferably wherein the antibody comprises or consists of the sequence set forth in SEQ ID NO:16.

In another embodiment, the antibody comprises a second antigen-binding region capable of binding human CD1d.

In a further embodiment, the antibody comprises a second antigen-binding region comprising the VH CDR1 sequence set forth in SEQ ID NO:17, the VH CDR2 sequence set forth in SEQ ID NO:18 and the VH CDR3 sequence set forth in SEQ ID NO:19.

In an even further embodiment, the antibody comprises a second antigen-binding region comprising the sequence set forth in SEQ ID NO:20, preferably wherein the antibody comprises or consists of the sequence set forth in SEQ ID NO:21.

In another embodiment, the antibody comprises a second antigen-binding region capable of binding a tumor antigen, wherein the tumor antigen is not human CD1d.

Tumor antigens are proteins that are produced by tumor cells that elicit an immune response, particularly T-cell mediated immune responses. The selection of the antigen binding moiety of the invention will depend on the particular type of cancer to be treated. Tumor antigens are well known in the art and include, for example a glioma-associated antigen, carcinoembryonic antigen (CEA), EGFRvIII, Interleukin-11 receptor alpha (IL-11Rα), Interleukin-13 receptor subunit alpha-2 (IL-13Ra or CD213A2), epidermal growth factor receptor (EGFR), B7H3 (CD276), Kit (CD117), carbonic anhydrase (CA-IX), CS-1 (also referred to as CD2 subset 1), Mucin 1, cell surface associated (MUC1), BCMA, oncogene fusion protein consisting of breakpoint cluster region (BCR) and Abelson murine leukemia viral oncogene homolog 1 (Abl) bcr-abl, Receptor tyro sine-protein kinase ERBB2 (HER2/neu), β-human chorionic gonadotropin, alphafetoprotein (AFP), anaplastic lymphoma kinase (ALK), CD19, CD123, cyclin BI, lectin-reactive AFP, Fos-related antigen 1, adrenoceptor beta 3 (ADRB3), thyroglobulin, tyrosinase; ephrin type-A receptor 2 (EphA2), Receptor for Advanced Glycation End products (RAGE-1), renal ubiquitous 1 (RU1), renal ubiquitous 2 (RU2), synovial sarcoma, X breakpoint 2 (SSX2), A kinase anchor protein 4 (AKAP-4), lymphocyte-specific protein tyrosine kinase (LCK), proacrosin binding protein sp32 (OY-TES1), Paired box protein Pax-5 (PAX5), Squamous Cell Carcinoma Antigen Recognized By T Cells 3 (SART3), C-type lectin-like molecule- 1 (CLL-1 or CLECL1), fucosyl GM1, hexasaccharide portion of globoH glycoceramide (GloboH), MN-CA IX, Epithelial cell adhesion molecule (EPCAM), EVT6-AML, transglutaminase 5 (TGS5), human telomerase reverse transcriptase (hTERT), polysialic acid, placenta-specific 1 (PLAC1), intestinal carboxyl esterase, LewisY antigen, sialyl Lewis adhesion molecule (sLe), lymphocyte antigen 6 complex, locus K 9 (LY6K), heat shock protein 70-2 mutated (mut hsp70-2), M-CSF, v-myc avian, myelocytomatosis viral oncogene neuroblastoma derived homolog (MYCN), Ras Homolog Family Member C (RhoC), Tyrosinase-related protein 2 (TRP-2), Cytochrome P450 1B1 (CYP1B1), CCCTC-Binding Factor (Zinc Finger Protein)-Like (BORIS or Brother of the Regulator of Imprinted Sites), prostate-specific antigen (PSA), paired box protein Pax-3 (PAX3), prostatic acid phosphatase (PAP), Cancer/testis antigen 1 (NY-ESO-1), Cancer/testis antigen 2 (LAGE-Ia), LMP2, neural cell adhesion molecule (NCAM), tumor protein p53 (p53), p53 mutant, Rat sarcoma (Ras) mutant, glycoprotein 100 (gp100), prostein, OR51E2, pannexin 3 (PANX3), prostate stem cell antigen (PSCA), high molecular weight-melanoma-associated antigen (HMWMAA), Hepatitis A virus cellular receptor 1 (HAVCRI), vascular endothelial growth factor receptor 2 (VEGFR2), Platelet-derived growth factor receptor beta (PDGFR-beta), legumain, human papilloma virus E6 (HPV E6), human papilloma virus E7 (HPV E7), survivin, telomerase, sperm protein 17 (SPA17), Stage-specific embryonic antigen-4 (SSEA-4), tyrosinase, TCR Gamma Alternate Reading Frame Protein (TARP), Wilms tumor protein (WT1), prostate-carcinoma tumor antigen- 1 (PCTA-1), melanoma inhibitor of apoptosis (ML-IAP), MAGE, Melanoma-associated antigen 1 (MAGE- Al), melanoma cancer testis antigen-1 (MAD-CT-1), melanoma cancer testis antigen-2 (MAD- CT-2), melanoma antigen recognized by T cells 1 (MelanA/MARTI), X Antigen Family, Member 1A (XAGE1), elongation factor 2 mutated (ELF2M), ERG (TMPRSS2 ETS fusion gene), N-Acetyl glucosaminyl-transferase V (NA17), neutrophil elastase, sarcoma translocation breakpoints, mammary gland differentiation antigen (NY-BR-1), ephrinB2, CD20, CD22, CD24, CD30, CD33, CD38, CD44v6, CD97, CD171, CD179a, androgen receptor, insulin growth factor (IGF)-I, IGF-II, IGF-I receptor, ganglioside GD2 (GD2), o-acetyl-GD2 ganglioside (OAcGD2), ganglioside GD3 (aNeu5Ac(2-8)aNeu5Ac(2-3)bDGalp(I-4)bDGlcp(I-I)Cer), ganglioside GM3 (aNeu5Ac(2-3)bDGa3p(I-4)bDGlcp(I-I)Cer), G protein-coupled receptor class C group 5, member D (GPRC5D), G protein-coupled receptor 20 (GPR20), chromosome X open reading frame 61 (CXORF61), folate receptor (FRα), folate receptor beta, Receptor tyrosine kinase-like orphan receptor 1 (ROR1), Fms-Like Tyrosine Kinase 3 (Flt3), Tumor-associated glycoprotein 72 (TAG72), Tn antigen (TN Ag or (GalNAca-Ser/Thr)), angiopoietin-binding cell surface receptor 2 (Tie 2), tumor endothelial marker 1 (TEM1 or CD248), tumor endothelial marker 7- related (TEM7R), claudin 6 (CLDN6), thyroid stimulating hormone receptor (TSHR), uroplakin 2 (UPK2), mesothelin, Protease Serine 21 (Testisin or PRSS21), epidermal growth factor receptor (EGFR), fibroblast activation protein alpha (FAP), Olfactory receptor 51E2 (OR51E2), ETS translocation-variant gene 6, located on chromosome 12p (ETV6-AML), CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module- containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); folate receptor (FRα); mesothelin; EGFR variant III (EGFRvIII); B-cell maturation antigen (BCMA); GD2; CLL-1; CA-IX; MUC1; HER2; Tumor-associated calcium signal transducer 2 (TROP2) and any combination thereof. In one preferred embodiment, the tumor antigen is selected from the group consisting of folate receptor (FRα), mesothelin, EGFRvIII, IL-13Ra, CD123, CD19, CD33, BCMA, GD2, CLL-1, CA-IX, MUC1, HER2, and any combination thereof. In one embodiment, the tumor target is PD-L1, EGFR, CD40, Her2, MUC-1, CEA, c-met, CD19, CD20, BCMA, Her3, AFP, CAIX, or CD38.

In a preferred embodiment of the method of the invention, the antibody comprises a second antigen-binding region capable of binding a tumor antigen selected from the group consisting of: human EpCAM, Her2, TROP2, CEA and MUC1.

In another embodiment of the method of the invention, the method does not lead to induction of the cytokine release syndrome, preferably the method does not lead to induction of IL-6 to levels above 10 ng/mL or above 2 ng/mL when determined as described in the Examples herein. In a further preferred embodiment, the method does not lead to induction of IL-2 to levels above 10 ng/mL or above 1 ng/mL when determined as described in the Examples herein. In a further preferred embodiment, the method does not lead to induction of MCP-1 to levels above 200 ng/mL or above 50 ng/mL when determined as described in the Examples herein.

As described above, in a further aspect, the invention relates to an antibody comprising an antigen-binding region capable of binding a human Vγ9Vδ2 T cell receptor for use in the treatment of cancer, wherein the treatment regimen comprises one or more administrations of a dose of at least 2 nmol/kg/day. Said antibody or said use may comprise any one of the further features mentioned herein for the method of treatment of the invention.

**Table 1: Sequence listing.**

| SEQ ID. | Code | Description | Sequence |
|---|---|---|---|
| 1 | 5C8 | CDR1 | NYAMG |
| 2 | 5C8 | CDR2 | AISWSGGSTSYADSVKG |
| 3 | 5C8 | CDR3 | QFSGADYGFGRLGIRGYEYDY |
| 4 | 6H4 | CDR1 | NYGMG |
| 5 | 6H4 | CDR2 | GISWSGGSTDYADSVKG |
| 6 | 6H4 | CDR3 | VFSGAETAYYPSDDYDY |
| 7 | 5C8 | VHH | EVQLVESGGGLVQAGGSLRLSCAASGRPFSNYA MGWFRQAPGKEREFVAAISWSGGSTSYADSVK GRFTISRDNAKNTVYLQMNSPKPEDTAIYYCAAQ FSGADYGFGRLGIRGYEYDYWGQGTQVTVSS |
| 8 | 5C8 variant 1 | Humanized sequence | EVQLLESGGGSVQPGGSLRLSCAASGRPFSNYA MSWFRQAPGKEREFVSAISWSGGSTSYADSVK GRFTISRDNSKNTLYLQMNSLRAEDTAVYYCAAQ FSGADYGFGRLGIRGYEYDYWGQGTQVTVSS |
| 9 | 5C8 variant 2 | Humanized sequence | EVQLLESGGGLVQPGGSLRLSCAASGRPFSNYA MSWFRQAPGKEREFVSAISWSGGSTSYADSVK GRFTISRDNSKNTLYLQMNSLRAEDTAVYYCAAQ FSGADYGFGRLGIRGYEYDYWGQGTLVTVSS |
| 10 | 6H4 | VHH | EVQLVESGGGLVQAGGSLRLSCAASGRPFSNYG MGWFRQAPGKKREFVAGISWSGGSTDYADSVK GRFTISRDNAKNTVYLQMNSLKPEDTAVYYCAAV FSGAETAYYPSDDYDYWGQGTQVTVSS |
| 11 | GS-linker | Linker 1 | GGGGS |
| 12 | 7D12 | CDR1 | SYGMG |
| 13 | 7D12 | CDR2 | GISWRGDSTGYADSVKG |
| 14 | 7D12 | CDR3 | AAGSAWYGTLYEYDY |
| 15 | 7D12 | VHH | EVQLVESGGGSVQTGGSLRLTCAASGRTSRSYG MGWFRQAPGKEREFVSGISWRGDSTGYADSVK GRFTISRDNAKNTVDLQMNSLKPEDTAIYYCAAA AGSAWYGTLYEYDYWGQGTQVTVSS |
| 16 | BsAb 7D12-5C8 | | EVQLVESGGGSVQTGGSLRLTCAASGRTSRSYG MGWFRQAPGKEREFVSGISWRGDSTGYADSVK GRFTISRDNAKNTVDLQMNSLKPEDTAIYYCAAA AGSAWYGTLYEYDYWGQGTQVTVSSGGGGS EVQLLESGGGSVQPGGSLRLSCAASGRPFSNYA MSWFRQAPGKEREFVSAISWSGGSTSYADSVK GRFTISRDNSKNTLYLQMNSLRAEDTAVYYCAAQ FSGADYGFGRLGIRGYEYDYWGQGTQVTVSS |
| 17 | 1D12 | CDR1 | DNVMG |
| 18 | 1D12 | CDR2 | TIRTGGSTNYADSVKG |
| 19 | 1D12 | CDR3 | TIPVPSTPYDY |
| 20 | 1D12 variant | Humanized sequence | EVQLVESGGGLVQAGGSLRLSCAASGSMFSDNV |
| | | | MGWYRQAPGKQRELVATIRTGGSTNYADSVKGR FTISRDNAKNTVYLQMNSLKPEDTAVYYCRHTIP VPSTPYDYWGQGTQVTVSS |
| 21 | BsAb 1D12-5C8 | | EVQLVESGGGLVQAGGSLRLSCAASGSMFSDNV MGWYRQAPGKQRELVATIRTGGSTNYADSVKGR FTISRDNAKNTVYLQMNSLKPEDTAVYYCRHTIP VPSTPYDYWGQGTQVTVSSGGGGSEVQLLESG GGSVQPGGSLRLSCAASGRPFSNYAMSWFRQAP GKEREFVSAISWSGGSTSYADSVKGRFTISRDN SKNTLYLQMNSLRAEDTAVYYCAAQFSGADYGF GRLGIRGYEYDYWGQGTQVTVSS |
| 22 | GS long linker | Linker 2 | GGGGSGGGGSGGGGSGGGGS |
| 23 | 7D12 variant | Humanized sequence | EVQLVESGGGSVQPGGSLRLSCAASGRTSRSYG MSWFRQAPGKEREFVSGISWRGDSTGYADSVK GRFTISRDNSKNTLYLQMNSLRAEDTAVYYCAAA AGSAWYGTLYEYDYWGQGTLVTVSS |

Antibodies used in the invention are typically produced recombinantly, i.e. by transfection of nucleic acid constructs encoding the antibodies in suitable host cells, followed by purification of the produced recombinant antibody from the cell culture medium. Nucleic acid constructs can be produced by standard molecular biological techniques well-known in the art. The constructs are typically introduced into the host cell using an expression vector. Suitable nucleic acid constructs and expression vectors are known in the art. Host cells suitable for the recombinant expression of antibodies are well-known in the art, and include CHO, HEK-293, Expi293F, PER-C6, NS/0 and Sp2/0 cells. Alternatively, expression may be performed in yeast such as Pichia pastoris, Hansenula polymorpha or Saccharomyces cerevisiae, or bacteria such as Escherichia coli.

Antibodies may be formulated with pharmaceutically-acceptable excipients in accordance with conventional techniques such as those disclosed in (Rowe et al., Handbook of Pharmaceutical Excipients, 2012 June, ISBN 9780857110275). The pharmaceutically-acceptable excipient as well as any other carriers, diluents or adjuvants should be suitable for the antibodies and the chosen mode of administration. Suitability of excipients and other components of pharmaceutical compositions is determined by the lack of significant negative impact on the desired biological properties of the chosen antibody or pharmaceutical composition of the present invention (e.g., less than a substantial impact (10% or less relative inhibition, 5% or less relative inhibition, etc.) upon antigen binding). A pharmaceutical composition may include diluents, fillers, salts, buffers, detergents (e.g., a nonionic detergent, such as Tween-20 or Tween-80), stabilizers (e.g., sugars or protein-free amino acids), preservatives, tissue fixatives, solubilizers, and/or other materials suitable for inclusion in a pharmaceutical composition. Further pharmaceutically-acceptable excipients include any and all suitable solvents, dispersion media, coatings, antibacterial and antifungal agents, isotonicity agents, antioxidants and absorption-delaying agents, and the like that are physiologically compatible with an antibody of the present invention.

### Examples

### Example 1 - Single Dose Pharmacokinetic and Multiple Dose Exploratory Toxicity in Female Cynomolgus Monkeys

### 1.1. Study Objective

This study objective was to determine the pharmacokinetics of single intravenous (IV) dosing and to evaluate the potential toxicity of four compounds via multiple IV doses in female cynomolgus monkeys.

### 1.2. Antibodies tested

Four different bispecific antibodies capable of binding the human Vγ9Vδ2 T cell receptor were tested. The Vγ9Vδ2 T cell receptor binding arm was based on antibody 7A5, a TCR Vy9-specific antibody (Janssen et al., J. Immunology 146(1) (1991), 35-39). Antibody 7A5 was tested for reactivity with both human-, as well as monkey (macaca fascicularis; cynomolgus monkey) Vγ9Vδ2 cells. Briefly, Vγ9Vδ2 cells were isolated by magnetic-activated cell sorting (MACS) and subsequently expanded from either human peripheral blood mononuclear cells (PBMC), or cynomolgus monkey PBMC. The first were isolated from the blood of a healthy volunteer and the latter were obtained from the BPRC (Rijswijk, the Netherlands). The protocol for isolation and expansion of these cells is described in the art (de Bruin et al., Clin. Immunology 169 (2016), 128-138; de Bruin et al., J. Immunology 198(1) (2017), 308-317). A serial dilution of the antibody was then tested for binding in FACS using pure (>95% Vγ9Vδ2 double-positive) polyclonal Vγ9Vδ2 cell populations and binding was detected with a FITC-conjugated anti-mouse antibody. The 7A5 antibody was found to be fully cross-reactive between the human- and cynomolgus monkey orthologs (data not shown). Therefore, the genes encoding the antibody were used for re-formatting into a Vγ9Vδ2 cell engager molecule.

The designed format of the bispecific Vγ9Vδ2 antibodies to be tested in the cynomolgus study was as follows: VHH against a tumor target (VHH: **v**ariable domain of the **h**eavy chain of **h**eavy-chain only antibodies)-linkerl-anti TCR Vγ9 7A5 scFv (VL-linker2-VH). The sequence of linker1 was GGGGS (SEQ ID NO:11). The sequence of linker2 was GGGGSGGGGSGGGGSGGGGS (SEQ ID NO: 22).

The cDNAs encoding these molecules were designed *in silico,* then ordered as synthetic genes at GeneArt (Thermo Fischer Scientific) and cloned for expression in an in-house generated expression vector. Protein was produced by transient transfection (and thereby expression) in HEK293E cells and purification from the culture supernatant by means of protein-A affinity chromatography, followed by preparative gel filtration and buffer exchange to phosphate-buffered saline (PBS) by U-Protein-Express, Utrecht, the Netherlands.

The affinity of the VHH-scFv fusions for human- and cynomolgus Vγ9Vδ2 cells was then determined using FACS staining essentially as described above, except for detection with a FITC-labeled polyclonal anti-llama IgG antibody. When mean fluorescence intensity (MFI) was plotted against the logarithm of the antibody concentration used, an S-shaped curve was obtained and the EC50 for binding was determined by curve fitting using GraphPad Prism software. The EC50 value for binding was 2-5nM for binding either to human- or cynomolgus monkey Vγ9Vδ2 cells, being not measurably different between the two orthologs.

### Antibody A: 1D22 x scFv 7A5

The first tumor target VHH that was fused with scFv 7A5 was 1D22 (VHH 22), a single domain antibody that binds human CD1d, described in WO16122320. The bispecific 1D22 x scFv 7A5 antibody made as fusion was tested for cross-reactivity with cynomolgus CD1d. Expression plasmids encoding either the human- or cynomolgus monkey CDld ortholog were generated. The CD1d-beta2 microglobulin (β2m) hetero-dimer was designed as a single-chain molecule with B2m at the N-terminus, spaced by a 15 amino acid glycine-serine stretch from the CDld molecule itself. The encoding cDNA was made as a synthetic gene and cloned into pCDNA3.1 as a HindIII-NotI fragment. Both expression constructs were then transiently transfected into HEK293T cells using polyethyleneimine (PEI), according to standard molecular and cell biological techniques. 24 hours after transfection, cells were used in FACS staining with the CDld targeting Vγ9Vδ2 cell engager 1D22 x scFv 7A5. A serial dilution of antibody was used to stain the transfected cells and staining was detected with a FITC-labeled polyclonal anti-lama IgG antibody. When MFI was plotted against the logarithm of the antibody concentration used, an S-shaped curve was obtained and the EC50 for binding was determined by curve fitting using GraphPad Prism software. The 1D22 x scFv 7A5 antibody bound both transfected cell populations with the same affinity: the EC50 was found to be 0.5nM for both.

### Antibody B: 7D12 x scFv 7A5

The second tumor target VHH that was fused with scFv 7A5 was 7D12 (SEQ ID NO:15), a single domain antibody that binds human EGFR. The bispecific 7D12 x scFv 7A5 antibody resulting from the fusion was functionally tested for cross-reactivity with cynomolgus EGFR. Recombinant, purified Epidermal Growth Factor (EGF) receptor (EGFR) extra-cellular domain (ECD) protein of either human- or cynomolgus origin (Sino Biologicals) was coated to the wells of a Maxisorb 96 well plate (Nunc) at 10 µg/ml in 50mM sodium carbonate buffer (pH 9). The following day, the EGFR coated plates were blocked with RPMI (Gibco) medium enriched with 10% Fetal calf serum (Hyclone). Human Vγ9Vδ2 cells were cultured according to previously published protocols (de Bruin et al., Clin. Immunology 169 (2016), 128-138; de Bruin et al., J. Immunology 198(1) (2017), 308-317). A concentration range of the 7D12 x scFv 7A5 antibody was then added to the wells in combination with 5x10E4 pure (>95% Vγ9⁺Vδ2⁺), expanded human Vγ9Vδ2 cells per well. In addition, to determine the level of degranulation and purity of the cells, differently labeled anti-CD107a-, anti-CD3- and anti-TCR Vγ9-antibodies were also included in the mix. Plates were then incubated for 4 hours at 37°C, 5% CO₂ and 80% humidity. After the incubation, cells were harvested and analyzed using flow cytometry to determine the level of CD107a expression on the Vγ9Vδ2 cells. The results were plotted as percentage of CD107a⁺ Vγ9Vδ2 cells as a function of the concentration of 7D12 x scFv 7A5 antibody used. Figure 1 shows that the 7D12 x scFv 7A5 antibody was equally potent in inducing human Vγ9Vδ2 cell degranulation dependent on either human-, or cynomolgus EGFR. This proves the anti-EGFR VHH used to be fully cross-reactive between the human- and cynomolgus ortholog.

To compare the potency of 7D12 x scFv 7A5 antibody in human Vγ9Vδ2 cell activation dependent on the human antigen to that in cynomolgus Vγ9Vδ2 cell activation dependent on the cynomolgus antigen, a similar assay as described above was performed. Human- as well as cynomolgus Vγ9Vδ2 cells were expanded as has been described (de Bruin et al., Clin. Immunology 169 (2016), 128-138; de Bruin et al., J. Immunology 198(1) (2017), 308-317) and were used in the assay together with human- or cynomolgus EGFR ECD protein (respectively). The results were plotted as percent of CD107a⁺ Vγ9Vδ2 cells as a function of the concentration of 7D12 x scFv 7A5 antibody used. Figure 2 shows 7D12 x scFv 7A5 antibody to display a differential, yet comparable potency when target and effector cells were matched.

### Antibody C: 2HCD25 x scFv 7A5

The third tumor target specific VHH that was used with scFv 7A5 was 2HCD25, a single domain antibody that binds human CD20, described in WO2017153402. Cross-reactivity to cynomolgus CD20 was evaluated by testing the ability of 2HCD25 - scFv 7A5 to induce human Vγ9Vδ2 cell degranulation in a human- as well as cynomolgus CD20-dependent way. Although there was a slight difference in potency, 2HCD25 was able to induce cynomolgus target-dependent human Vγ9Vδ2 cell activation with pM potency (data not shown).

### Antibody D: 1D12 x scFv 7A5

The fourth tumor target specific VHH that was fused with scFv 7A5 was 1D12 (VHH 12), a single domain antibody that binds human CD1d, described in WO16122320. The bispecific 1D12 x scFv 7A5 antibody does not cross-react with cynomolgus CDld (data not shown). This bispecific antibody is functionally monovalent in monkey and was used as a Vγ9Vδ2-only binding control.

### 1.3 Experimental Design

Non-human primate (NHP) care and use was conducted in accordance with all Applicable Assessment and Accreditation of Laboratory Animal Care (AAALAC) regulations and guidelines.

Antibodies A and B was tested in a first study and antibodies C and D were tested in a second study. For each of these studies, a total of 12 female NHPs with weight 3.0 ∼ 5.0 kg were enrolled for the two antibodies tested. Each study was divided into 2 parts: phase 1 (the pharmacokinetics of single IV dose, 3*NHPs/compound, N=1, group 1-6) and phase 2 (the potential toxicity of multiple IV dose, 3*NHPs/compound, N=1, group 7-12). The phase 2 was initiated after the PK bleeding from phase 1. The study design and animal grouping information for compounds A and B are summarized as Table 2. Study design and animal grouping information for compounds C and D were as for compound A. Monkeys in phase 1 received a single dose (SD) of drug administration and monkeys in phase 2 received 7 times of drug administration.

**Table 2:**

| **Phase** | **Group** | **Test Ab** | **Dosage (mg/kg)** | **Dosing Route** | **Dose frequency** | **Necropsy** |
|---|---|---|---|---|---|---|
| Phase 1 | 1 | **B** | 0.03 | IV | SD | No necropsy |
| | 2 | | 0.1 | IV | SD | No necropsy |
| | 3 | | 0.3 | IV | SD | No necropsy |
| | 4 | **A** | 0.1 | IV | SD | No necropsy |
| | 5 | | 0.3 | IV | SD | No necropsy |
| | 6 | | 1.0 | IV | SD | No necropsy |
| Phase 2 | 7 | **B** | 0.03 | IV | 7x daily | 1 day after last dose |
| | 8 | | 0.1 | IV | 7x daily | 1 day after last dose |
| | 9 | | 0.3 | IV | 7x daily | 1 day after last dose |
| | 10 | **A** | 0.1 | IV | 7x daily | 1 day after last dose |
| | 11 | | 0.3 | IV | 7x daily | 1 day after last dose |
| | 12 | | 1.0 | IV | 7x daily | 1 day after last dose |

| | | | | | | |
|---|---|---|---|---|---|---|
| Note: The testing compounds in phase 1 were dosed in parallel at 3-day intervals. | | | | | | |

The dose levels of compounds in phase 2 were determined based on the outcome of phase 1. The animals in phase 2 were dosed on the same day.

### 1.4 Experimental procedures

**Drug administration:** The testing compounds were administrated via 30-min intravenous infusion at a rate of 5 mL/kg for each testing compound per administration as noted in the table 2. The infusion site was marked.

**Clinical observation:** During the study, clinical observations, such as animal's activity, behavior, appetite, vomiting, and stools, were conducted. Any abnormalities observed were reported to the veterinarian, study director and sponsor and recorded.

The detailed of blood collection for groups 7-12 is summarized in Table 3.

**Table 3: Blood sampling information for groups 7-12**

| Time point | Hematology | Coagulation | Clin Chemistry | Flow cytometry | Cytokines |
|---|---|---|---|---|---|
| Pre-dose | XX | XX | XX | XX | XX |
| Day 0 | | | | | |
| 30 min | | | | | X |
| 2 hrs | X | | | X | X |
| 4 hrs | | | | | X |
| 8 hrs | | | | | X |
| Day 1 | | | | | |
| Pre-dose | X | | | X | X |
| 2 hrs | X | | | X | X |
| Day 2 | | | | | |
| Pre-dose | X | | X | X | X |
| 2 hrs | X | | | X | X |
| Day 3 | | | | | |
| Pre-dose | X | X | | X | X |
| 2 hrs | X | | | X | X |
| Day 4 | | | | | |
| Pre-dose | X | | | X | X |
| 2 hrs | X | | | X | X |
| Day 5 | | | | | |
| Pre-dose | X | | X | X | X |
| 2 hrs | X | | | X | X |
| Day 6 | | | | | |
| Pre-dose | X | | | X | X |
| 30 min | | | | | X |
| 2 hrs | X | | | X | X |
| 4 hrs | | | | | X |
| 8 hrs | | | | | X |
| Day 7 | | | | | |
| 23 hrs | X | X | X | X | X |

| | | | | | |
|---|---|---|---|---|---|
| **Hematology:** For the groups 7-12 only (Table 3). Whole blood (1 mL) was collected into pre-labelled BD Vacutainer® K2-EDTA tube (367841) and kept on ice until being analyzed for complete blood cell count (CBC). **Coagulation test:** For the groups 7-12 only (Table 3). Whole blood (1.8 mL) was collected into Vacuette® PREMIUM tube with 3.8% buffered sodium citrate solution (454381). The whole blood samples were stored at 4 degree or wet ice at all times until analysis for prothrombin time (PT), activated partial thromboplastin time (APTT), fibrinogen (FIB) and thrombin time (TT). **Clinical chemistry:** For the groups 7-12 (Table 3). The animals were fasted overnight for clinical chemistry assays. Blood sample (1.0 to 2.0 mL) was collected from a cephalic or saphenous vein into an appropriate pre-labeled BD Vacutainer® SST II Plus plastic serum tube (367957) for liver function (e.g. alanine aminotransferase (ALT), aspartate aminotransferase (AST), total bilirubin (TBil), direct bilirubin (DB), total protein (TP), prealbumin (PA), albumin (ALB), globulin (GLB), albumin/globulin (A/G) ratio, alkaline phosphatase (ALP), Gamma Glutamyl Transferase (γ-GT), adenosine deaminase (ADA), lactate dehydrogenase (LDH)) and kidney function (eg. blood urea nitrogen (BUN), serum creatinine (Scr), blood uric acid (UA), cystatin (C) measurements. The collected blood samples were allowed to clot for a minimum of 30 minutes at room temperature and then centrifuged at 3,500 rpm for 10 minutes. The serum samples were stored at 4°C or on wet ice for clinical chemistry assay on the same day of sample collection. **Flow cytometry:** Flow cytometry tests were performed twice during 2-week training period as the baseline for both phases. In addition, 2-hour post-dose and thereafter daily up to 7 days for the groups 1∼6 and 2 hours and 24 hours (prior to the next dosing) after completion of each infusion for the groups 7-12 (Table 3). Whole blood (0.9 mL) was collected into a pre-labelled BD Vacutainer® K2-EDTA tube (367841) and kept on ice until being transferred to Genomics & Biomarker department of Crown Bioscience (Taicang) Inc. for further analysis (big panel (CD45, CD3, CD4, CD8, CD14, CD16, CD20, CD25, CD27, CD69, CD107a, PD-1, TCR Vgamma9 and TCR γ/δ) and additional small panel stained with CD3, TCR Vgamma9 and anti-VHH antibody only.) **Plasma cytokine analysis:** For the groups 7-12 only. Blood samples (0.7 mL) were collected into pre-labelled BD Vacutainer® K2-EDTA tube (367841) and kept on ice or wet ice all the time before centrifugation. Blood sample tubes were centrifuged at 4°C, 3000rpm for 10 minutes; immediately after centrifugation, the plasma was transferred into pre-labeled polypropylene screw-cap vials and stored in a freezer at -80°C until being analyzed (IL-1β, IL-2, IL-4, IL-5, IL-6, IL-8, IL-10, TNF-α, IFN-γ, Il-12p70, IL-15, CCL2). | | | | | |

The time points for cytokine analysis were: two times baseline (pre-dose) during 2-week training period, and then 0.5, 2, 4 and 8 hours after completion of infusion on day 0, prior to the dosing and then 2 hours after completion of infusion on day 1∼5 and prior to the dosing and then 0.5, 2, 4, 8 and 24 hours after completion of infusion on day 6.

### 1.5 Results

### 1.5.1 Clinical signs

No clinical signs were detected for any of the antibodies at any dose. Antibodies **A** (1D22 x scFv 7A5), **C** (2HCD25 x scFv 7A5) and **D** (1D12 x scFv 7A5) could be dosed at least to 1 mg/kg/day (30 min infusion) without clinical signs in a multiple dose setting (7 doses on 7 consecutive days). Dosing of up to 33 nmol/kg/day or 66 nmol/kg/hr once daily during 7 consecutive days therefore did not lead to clinical signs.

Antibody **B** (7D12 x scFv 7A5) could be dosed at least to 0.3 mg/kg/day (30 min infusion) without clinical signs in a multiple dose setting (7 doses on 7 consecutive days). Dosing of up to 10 nmol/kg/day or 20 nmol/kg/hr once daily during 7 consecutive days therefore did not lead to clinical signs.

### 1.5.2 Detection of VHH coated Vy9 cells in non-human primates after a single i.v. infusion

In phase 1 of the study, monkeys were given a single i.v. infusion of the antibody **D** (1D12 x scFv 7A5) molecule at doses of 0.1mg/kg/day, 0.3mg/kg/day and 1mg/kg/day. Each dose was given as a half hour infusion. In order to avoid tumor-target-related effects, the CD1d-specific VHH chosen for this analysis was the 1D12-based antibody fragment that is not cross-reactive to the cynomolgus ortholog. A blood sample was taken from the animals at different time intervals, i.e. 2 hours after infusion and subsequently daily for seven consecutive days. Using CD3 and Vγ9 staining (with a non-competitive Vγ9 specific antibody) as markers for Vγ9 cells, the presence of VHH on Vγ9 cells was assessed by flow cytometry using an AF647-labelled rabbit monoclonal anti-VHH antibody. Figure 3 shows the percentage of Vγ9 cells (defined as described above) scoring positive for VHH labeling as a function of time after a single i.v. infusion of antibody. Independent of the dose given, all Vγ9 cells were scored positive for VHH labeling 2 hours after the infusion, whereas the detection of VHH on Vy9 cells was dose-dependent on day 1. On day 2, the presence of VHH could only be detected on Vγ9 cells in the animal receiving the highest dose of antibody.

### 1.5.3 CD20 binding antibody induced a dose-dependent B cell depletion in cynomolgus monkey after multiple dose injection

In phase 2 of the study, the cynomolgus monkey cross-reactive, CD20-targeting compound antibody **C** (2HCD25 x scFv 7A5) and the CDld targeting, non-cynomolgus cross-reactive antibody **D** (1D12 x scFv 7A5) were used in a multiple dose toxicity study in cynomolgus monkeys. Animals were given daily doses of 0.1mg/kg, 0.3mg/kg and 1mg/kg in a half hour infusion. Blood was taken from the animals daily and the percentage of B-cells in the periphery was determined by staining for CD20 (in combination with CD21 and CD27). Figure 4 shows the relative number of B-cells (as a percentage of the number found before the first dose was administered) as a function of time for an animal receiving 2HCD25 x scFv 7A5 (upper panel) or an animal treated with 1D12 x scFv 7A5 (lower panel). The figure shows 2HCD25 x scFv 7A5 to induce a dose-dependent decrease in the percentage of B-cells found in peripheral blood, in contrast to 1D12 x scFv 7A5 (i.e. the Vγ9Vδ2-only binding control) that had no significant and consistent effect on the number of B-cells.

### 1.5.4 Multiple dose injection of 2HCD25 x scFv 7A5, but not 1D12 x scFv 7A5 in cynomolgus monkey causes a very mild cytokine release only after

**the first injection.** During phase 2 of the study, cytokine levels in plasma were determined after every injection. Figure 5 shows that there was a very mild cytokine release (shown here for IL-6) only observed after the first injection with antibody **C** (2HCD25 x scFv 7A5) but not after injection with control antibody **D** (1D12 x scFv 7A5) (not shown). This was consistently seen for several cytokines (e.g. IL-2, MCP-1) Mild cytokine release was also observed after a first dose of antibody **A** (1D22 x scFv 7A5) and antibody **B** (7D12 x scFv 7A5). The very mild cytokine releases are consistent with the absence of clinical signs.

### 1.5.5 Coagulation, clinical chemistry and haematology observations

### Coagulation test

There were no significant changes observed on PT, APTT, TT and FIB during the multiple dose study.

### Clinical chemistry

No dose-related changes were found during the study for liver function (ALT, AST, TBil, DB, TP, PA, ALB, GLB, A/G ratio, ALP, γ-GT, ADA, LDH) and kidney function (BUN, Scr, UA, cystatin C) measurements in the multiple dose study.

## Claims

1. A method for the treatment of cancer, comprising administration of an antibody capable of binding a human Vγ9Vδ2 T cell receptor to a human subject in need thereof, wherein the treatment regimen comprises one or more administrations of a dose of at least 2 nmol/kg/day.

2. The method according to claim 1, wherein the treatment regimen comprises one or more administrations of a dose of at least 2.5 nmol/kg/day, e.g. a dose of at least 5 nmol/kg/day, such as a dose of at least 10 nmol/kg/day, e.g. a dose of at least 25 nmol/kg/day, such as a dose of at least 50 nmol/kg/day, e.g. a dose of at least 100 nmol/kg/day.

3. The method according to claim 1 or 2, wherein said administration is carried out intravenously over a time period of between 0.5 and 8 hours, such as between 1 and 8 hours, e.g. between 2 and 6 hours or between 1 and 4 hours.

4. The method according to claim 1 or 2, wherein said administration is carried out subcutaneously.

5. The method according to any one of the preceding claims, wherein said dose is administered more than once, preferably wherein the time interval between each of the administrations is from 7 to 21 days, e.g. wherein administration is twice weekly, weekly or once every two weeks.

6. The method according to any one of the preceding claims, wherein the antibody is capable of activating human Vγ9Vδ2 T cells.

7. The method according to any one of the preceding claims, wherein the antibody capable of binding a human Vγ9Vδ2 T cell receptor comprises or consists of a single-domain antibody.

8. The method according to any one of the preceding claims, wherein the antibody has a K_{D} for Vγ9Vδ2 T cell receptor binding of 10⁻⁸ M or less, such as 10⁻⁹ M or less or 10⁻¹⁰ M or less.

9. The method according to any one of the preceding claims, wherein the antibody is capable of binding to human Vδ2.

10. The method according to any one of the preceding claims, wherein the antibody competes for binding to human Vδ2 with an antibody having the sequence set forth in SEQ ID NO: 8 or competes for binding to human Vδ2 with an antibody having the sequence set forth in SEQ ID NO: 10, e.g. wherein the antibody binds the same epitope on human Vδ2 as an antibody having the sequence set forth in SEQ ID NO: 8 or binds the same epitope on human Vδ2 as an antibody having the sequence set forth in SEQ ID NO: 10.

11. The method according to any one of the preceding claims, wherein the antibody comprises an antigen-binding region comprising the VH CDR1 sequence set forth in SEQ ID NO:1, the VH CDR2 sequence set forth in SEQ ID NO:2 and the VH CDR3 sequence set forth in SEQ ID NO:3 or comprises the VH CDR1 sequence set forth in SEQ ID NO:4, the VH CDR2 sequence set forth in SEQ ID NO:5 and the VH CDR3 sequence set forth in SEQ ID NO:6.

12. The method according to any one of the preceding claims, wherein the antibody comprises or consists of a sequence selected from the group consisting of: SEQ ID NO: 7, 8, 9 and 10.

13. The method according to any one of the preceding claims, wherein the antibody capable of binding a human Vγ9Vδ2 T cell receptor is a multispecific antibody having a (first) antigen-binding region capable of binding human Vγ9Vδ2 T cell receptor and a second antigen-binding region capable of binding a tumor antigen.

14. The method according to any claim 13, wherein the multispecific antibody is a bispecific antibody.

15. The method according to 13 or 14, wherein the second antigen-binding region comprises or consists of a single-domain antibody.

16. The method according to any one of the preceding claims, wherein the method is for the treatment of a solid tumor cancer and wherein the antibody comprises a second antigen-binding region capable of binding a target expressed by solid tumors.

17. The method according to any one of the preceding claims, wherein the antibody comprises a second antigen-binding region capable of binding human EGFR.

18. The method according to any one of the preceding claims, wherein the antibody comprises a second antigen-binding region comprising the VH CDR1 sequence set forth in SEQ ID NO:12, the VH CDR2 sequence set forth in SEQ ID NO:13 and the VH CDR3 sequence set forth in SEQ ID NO:14.

19. The method according to any one of the preceding claims, wherein the antibody comprises a second antigen-binding region comprising the sequence set forth in SEQ ID NO:15 or SEQ ID NO:23, preferably wherein the antibody comprises or consists of the sequence set forth in SEQ ID NO:16.

20. The method according to any one of claim 1 to 15, wherein the antibody comprises a second antigen-binding region capable of binding human CD1d.

21. The method according to any one of the preceding claims, wherein the antibody comprises a second antigen-binding region comprising the VH CDR1 sequence set forth in SEQ ID NO:17, the VH CDR2 sequence set forth in SEQ ID NO:18 and the VH CDR3 sequence set forth in SEQ ID NO:19.

22. The method according to any one of the preceding claims, wherein the antibody comprises a second antigen-binding region comprising the sequence set forth in SEQ ID NO:20, preferably wherein the antibody comprises or consists of the sequence set forth in SEQ ID NO:21.

23. The method according to any one of the preceding claims, wherein the antibody comprises a second antigen-binding region capable of binding a tumor antigen, wherein the tumor antigen is not human CD1d.

24. The method according to any one of claim 1 to 15, wherein the antibody comprises a second antigen-binding region capable of binding a tumor antigen selected from the group consisting of: EpCAM, Her2, TROP2, CEA and MUC1.

25. An antibody comprising an antigen-binding region capable of binding a human Vγ9Vδ2 T cell receptor for use in the treatment of cancer, wherein the treatment regimen comprises one or more administrations of a dose of at least 2 nmol/kg/day.

26. The antibody for use in the treatment of cancer according to claim 25, wherein the antibody or the use comprises the features set forth in any of claims 2 to 24.
